# EUROPEAN PATENT APPLICATION

(11) **EP 4 019 944 A1**
(43) Date of publication of application: **29.06.2022**
(21) Application number: 20217011.4
(22) Date of filing: 23.12.2020
(51) Int. Cl.: G01N 21/31, G01J 3/00, G01N 21/85, G01N 33/18

(54) **MULTI-WAVELENGTH PROCESS PHOTOMETER**

(71) Applicant: Hach Lange GmbH, 14163 Berlin (DE)
(72) Inventor: Mitreiter, Andreas, 14163 Berlin (DE); Haase, Barbara, 14163 Berlin (DE); Naggies, Andrè, 14163 Berlin (DE)
(74) Representative: terpatent Patentanwälte ter Smitten Eberlein-Van Hoof Rütten Daubert

(57) **Abstract**

The invention is directed to a multi-wavelength process photometer (20) for quasi-continuously determining the absorption of a liquid sample, comprising
a continuous-spectrum flashlight source (24),
a transparent liquid sample measurement cell (40) which is radiated by the flashlight source (24),
a translucent light diffusor element (50) behind the measurement cell (40) for homogenously diffusing the light of the flashlight source (24) coming from the liquid sample measurement cell (40), and
at least two different wavelength-selective light detectors (61,62,63) behind the light diffusor element (50), wherein the light detectors (61,62,63) have substantially the same distance (X4) to the light diffusor element (50).

## Description

The present invention refers to a multi-wavelength process photometer for quasi-continuously determining the absorption of a liquid sample, and to a wastewater measurement arrangement with an immersion probe immersed into wastewater of a wastewater tank, the immersion probe comprising the multi-wavelength process photometer.

A process photometer can quasi-continuously determine the light absorption of a liquid sample at different wavelengths. The liquid sample can, for example, be wastewater in a wastewater tank of a wastewater treatment plant. The process photometer thereby provides real-time actual analyte concentration values in a closed loop control circuit. The process photometer determines the absorption of the liquid sample at different wavelengths for simultaneously determining the concentration values of different analytes and/or for improving the measurement quality.

State of the art process photometers are provided with a continuous-spectrum flashlight source where the flashlight is directed to a sample measurement cell, and with sophisticated optical arrangements to provide that all different wavelength-selective light detectors receive the flashlight coming from the sample measurement cell with exactly the same intensity at exactly the same time. These optical arrangements are generally mechanically sensitive, require high quality optical components, and need to be precisely adjusted to provide that all different wavelength-selective light detectors receive the flashlight coming from the measurement cell with exactly the same intensity. If the liquid sample is turbid, the measurement results can be significantly falsified.

It is an object of the invention to provide a simple and robust multi-wavelength process photometer.

This object is solved with a multi-wavelength process photometer with the features of main claim 1.

The multi-wavelength process photometer according to the invention is suitable for quasi-continuously determining the extinction or the absorption of a liquid sample simultaneously at different wavelengths. The process photometer is provided with a continuous-spectrum flashlight source, which can, for example, be a xenon flash lamp with a sufficiently continuous light emitting spectrum. The flashlight-source can be intermittently activated with a frequency of, for example, 1 to 50 Hz. The multi-wavelength process photometer is not necessarily used in a process, but is generally suitable to be used in a process because the photometer can quasi-continuously provide photometric measurements at different wavelengths simultaneously.

The process photometer is provided with a transparent liquid sample measurement cell which is radiated by the flashlight source. The liquid sample measurement cell is preferably a flow cell through which the liquid sample continuously flows through, for example, pumped by an electric sample liquid pump. The high transparency of the measurement cell allows the light of the flashlight source to radiate through the measurement cell, including the liquid sample volume therein, to thereby allow a determination of the extinction/absorption of the liquid sample within the sample measurement cell to be made. The transparency of the sample measurement cell walls should be as large as possible for the relevant wavelength spectrum of the flashlight source.

As seen from the flashlight source, a translucent light diffusor element is provided behind the measurement cell for homogeneously diffusing the light of the flashlight source coming from the liquid sample measurement cell. The light diffusor element highly diffuses the light coming from the sample measurement cell so that a homogeneous, non-concentrated, and diffuse light cone is generated by the light diffusor element.

The light diffusor element generates a spatially homogeneous light cone with a homogeneity cone angle of at least a few degrees, preferably of a homogeneity cone angle of at least 15° to 40°. The larger the homogeneity cone angle is, the closer are the light detectors arranged to the light diffusor element. The closer the light detectors are arranged to the light diffusor element, the higher is the signal to noise ratio of the light signal received by the light detectors. The spatial homogeneity of the wavelength spectrum and of the light intensity is sufficient within the homogeneity cone angle to have exactly the same spectrum and intensity at a measurement plane being substantially parallel to the preferably plane light diffusor element.

At least two different wavelength-sensitive light detectors are provided behind the light diffusor element within the homogeneity cone angle. The at least two light detectors all have substantially the same distance to the light diffusor element.

Using a translucent light diffusor element in combination with two or more wavelength-sensitive light detectors having an identical distance with respect to the light diffusor element and lying within the homogeneity cone angle provides a highly reliable and mechanically very robust arrangement which allows a simultaneous determination of the absorption/extinction of the liquid sample at two, three, or more different wavelengths. No high precision optical elements are thereby required because a very precise adjustment of the optical elements is no longer necessary for assembling the multi-wavelength process photometer. A turbid liquid sample does not effect the measurement results.

A converging lens is preferably arranged between the flashlight source and the sample measurement cell. The converging lens focuses the light of the flashlight source at the or within the measuring section of the liquid sample measurement cell. The converging lens concentrates as much of the flashlight source light as possible within the measuring section of the sample measurement cell so that a relatively high signal to noise ratio is realized.

According to a preferred embodiment, the light diffusor element is defined by a translucent diffusor body with micro-inclusions of less than 30 µm diameter, preferably of less than 8,0 µm diameter. The maximum diameter of the micro-inclusions is adapted to the relevant wavelength measured by the wavelength-selective light detectors. The micro-inclusions can, for example, be micro-bubbles of gas.

The light diffusor element is preferably defined by a translucent diffusor body with a concentration of micro-inclusions of more than 100 mio/cm³ and less than 5000 mio/cm³, preferably of less than 2500 mio/cm³, and with an effective optical thickness of 0.5 to 5.0 mm. This micro-inclusions concentration provides sufficient diffusion within a relatively thin and plate-like diffusor body.

The detector-facing surface of the light diffusor element preferably has a light scattering surface structure. The scattering surface structure has a structure fineness that provides that light impinging the surface structure is diffusely reflected. A typical wavelength-selective light detector is provided with a photosensitive semiconductor and a holographic wavelength-selective filter which is perfectly plane. Since the holographic wavelength-selective filter surface is plane, the filter reflects a fraction of the incoming light backwards to the light diffusor element which again reflects this fraction back to the light detectors. Since the detector-facing surface of the light diffusor element scatters the light reflected by the filter elements, this reflected light fraction does not relevantly affect the absorption measurement.

The process photometer preferably comprises an electronic photometer control with a summarizing module for summarizing the measurement signals of the light detectors of at least 10 light flashes of the flashlight source. The signal to noise ratio of the light received by the wavelength-selective light detectors is relatively low because of the intentionally high light diffusion quality of the light diffusor element. The use of the signal-weakening light diffusor element is compensated by integrating 10 or even more light/signals in the summarizing module.

According to a preferred embodiment, at least three wavelength-sensitive light detectors are provided, each having a filtering wavelength of between 195 and 240 nm. The three wavelength-sensitive light detectors can, for example, have a filter wavelength of 205, 215 and 230 nm for detecting nitrate, nitrite, and a reference or another component of the absorbtion matrix. The light detector with the reference wavelength is used for having a reference of the general intensity of the received light signal.

According to a preferred embodiment, a wastewater process measurement arrangement is provided with an immersion probe immersed into wastewater of a wastewater tank. The immersion probe comprises the multi-wavelength process photometer of one of claims 1 to 7. Since the multi-wavelength process photometer is provided within or as a part of the immersion probe, no pumping of the liquid sample to a land-based photometer is needed, so that a delay between the sampling action and the absorption measurement is avoided and the reaction time of a closed loop control circuit is minimized.

One embodiment of the invention is described with reference to the enclosed drawings, wherein:
Figure 1 schematically shows a wastewater measurement arrangement with an immersion probe immersed into wastewater, the immersion probe comprising a multi-wavelength process photometer according to the invention; and
Figure 2 schematically shows in more detail the multi-wavelength process photometer of Figure 1.

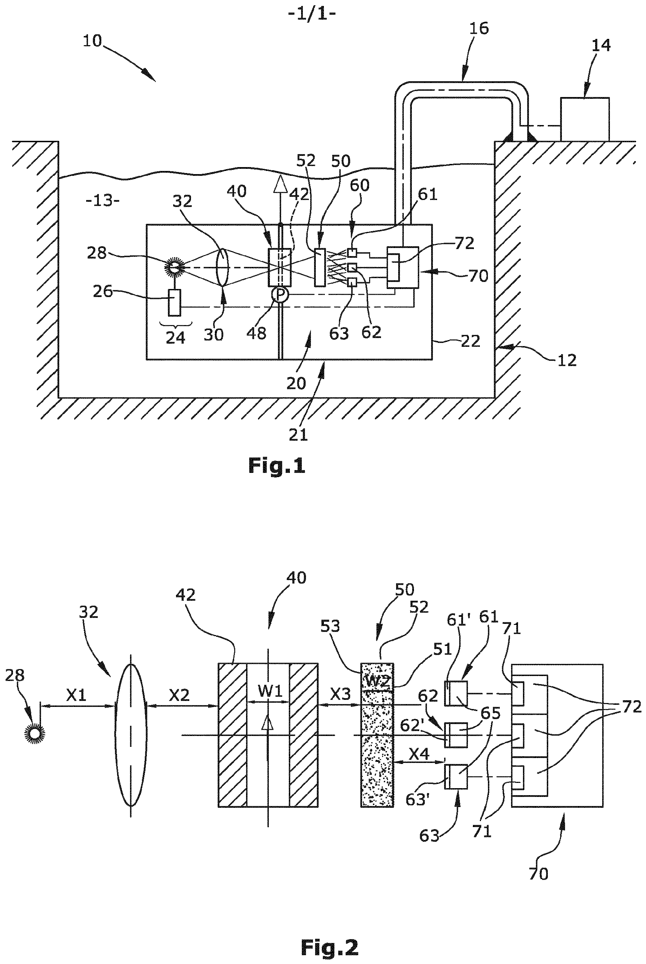

Figure 1 schematically shows a wastewater measurement arrangement 10 being a part of a wastewater treatment plant for cleaning wastewater. The wastewater measurement arrangement 10 is provided with a large wastewater tank 12 with wastewater 13. The wastewater tank 12 is a tank in a series of wastewater treatment tanks (which is not shown). The wastewater measurement arrangement 10 comprises an immersion probe 21 which is immersed into the wastewater 13 and which is held by a stationary holding arm 16. The immersion probe 21 comprises a multi-wavelength process photometer 20 which quasi-continuously determines, in real-time, the nitrite quantity of the wastewater 13 and which is electrically connected to a land-based control device 14 via suitable energy lines and signal lines.

The process photometer 20 is provided with a continuous-spectrum flashlight source 24 which comprises a flashlight energy generator 26 for generating electric energy pulses and a xenon illuminant 28. The xenon illuminant 28 generally has a long life and generates a continuous wavelength spectrum. The flashlight energy generator 26 can generate electric energy pulses with a frequency of up to 20 Hz. The flashlight source 24 generates light pulses with a sufficiently continuous wavelength spectrum, in particular in the ultraviolet range of 150 to 300 nm.

The process photometer 20 is provided with a converging lens 30 which is arranged in-line with and between the flashlight source 24 and a transparent sample measurement cell 40 which is a flow cell. The lens body 32 of the converging lens 30 is bi-convex and concentrates the light of the flashlight source 24 within a measuring section 42 of the sample measurement cell 40. The axial optical length W1 of the measuring section 42, which is the transverse diameter of the liquid line, is 2.0 mm in the present embodiment. The measurement cell 40 can be provided with a wiper for cleaning the inner surfaces of the liquid line in the measuring section 42. A liquid sample of the wastewater 13 is continuously pumped by an electric sample pump 48 to the measuring section 42 of the measurement cell 40. The body of the measurement cell 40 is substantially transparent for the light radiated by the flashlight source 24 and is concentrated by the converging lens 30 at the measuring section 42.

A translucent light diffusor element 50 is provided, as seen from the flashlight source 24, behind the sample measurement cell 40. The light diffusor element 50 is defined by a translucent and plate-like diffusor body 52 with micro-inclusions with a typical inclusion diameter of 3-4 µm, with an inclusions concentration of 300 mio/cm³ and with an axial optical thickness W2 of about 1.0 mm. The diffusor element 50 generates a spatially homogeneous light cone with a total cone angle of approximately 30°. The detector-facing surface 52 of the light diffusor element 50 has a light scattering surface.

Three different wavelength-selective light detectors 61,62,63 are arranged behind the light diffusor element 50. The three light detectors 61,62,63 all have substantially the same distance X4 to the light diffusor element 50. Every light detector 61,62,63 is provided with a silicon photo sensor 65 and with an individual interference filter 61',62',63' of filter wavelengths of 215 nm, 205 nm and 230 nm, respectively. The present light detector arrangement allows a precise determination the concentration of nitrate to be made, whereas the 215 nm detector 61 determines the absorption caused by nitrite, the 205nm detector 62 determines the absorption caused by nitrate and nitrite, and the 230 nm-detector 63 determines the general light intensity of the flashlight source 24 and, if given, other components of the absortion matrix.

The signals of the photo sensors 65 are amplified by three signal amplifier modules 71, and 50 consecutive amplified signals are summarized by three summarizing modules 72 to thereby provide a measurement result with a high signal to noise ratio. The light detectors 61,62,63 are part of an electronic photometer control 70 which also controls and synchronizes the flashlight source 24, which controls the sample pump 48, and which communicates with the land-based control device 14.

In the present embodiment of the process photometer 20, the distance X1 between an aperture of the flashlight source 24 and the convex light inlet surface of the converging lens 30 is about 17 mm, the distance X2 between the convex outlet surface of the converging lens 30 and the plane inlet surface of the measurement cell 40 is about 7.5 mm, the distance X3 between the outlet surface of the measurement cell 40 and the light diffusor element 50 is 13.5 mm, and the distance X4 between the light diffusor element 50 and the light detectors is 17.1 mm. The focal length of the converging lens 30 is about 9 mm so that the focus of the light generated by the flashlight source 24 lies in the center of the measuring section 42.

## Claims

1. A multi-wavelength process photometer (20) for quasi-continuously determining an absorption of a liquid sample, the multi-wavelength process photometer (20) comprising:
a continuous-spectrum flashlight source (24);
a transparent liquid sample measurement cell (40) which is radiated by the continuous-spectrum flashlight source (24);
a translucent light diffusor element (50) which is arranged behind the transparent liquid sample measurement cell (40) for homogenously diffusing the light of the continuous-spectrum flashlight source (24) coming from the transparent liquid sample measurement cell (40); and
at least two different wavelength-selective light detectors (61,62,63) which are arranged behind the translucent light diffusor element (50), the at least two different wavelength-selective light detectors (61,62,63) having substantially a same distance (X4) to the translucent light diffusor element (50).

2. The multi-wavelength process photometer (20) of claim 1, further comprising:
a converging lens (30) which is arranged between the continuous-spectrum flashlight source (24) and the transparent liquid sample measurement cell (40), the converging lens (30) focusing the light of the continuous-spectrum flashlight source (24) at a measuring section (42) of the transparent liquid sample measurement cell (40).

3. The multi-wavelength process photometer (20) of one of the preceding claims, wherein the translucent light diffusor element (50) is defined by a translucent diffusor body (52) with micro-inclusions of less than 30 µm diameter, preferably of less than 8,0 µm diameter.

4. The multi-wavelength process photometer (20) of one of the preceding claims, wherein the translucent light diffusor element (50) is defined by a translucent diffusor body (52) with a concentration of micro-inclusions of more than 100 mio/cm³ and less than 5000 mio/cm³, preferably less than 2500 mio/cm³, and with an effective optical thickness (W2) of 0.5 to 5.0 mm.

5. The multi-wavelength process photometer (20) of one of the preceding claims, wherein the translucent light diffusor element (50) has a detector-facing surface (51) which has a light scattering surface structure.

6. The multi-wavelength process photometer (20) one of the preceding claims, further comprising:
an electronic photometer control (70) with a summarizing module (72) for summarizing measurement signals of the at least two different wavelength-selective light detectors (61, 62, 63) of at least 10 light flashes of the continuous-spectrum flashlight source (24).

7. The multi-wavelength process photometer (20) of one of the preceding claims, wherein at least three of the at least two different wavelength-selective light detectors (61,62,63) are provided, each of which have a filtering wavelength of between 195 and 240 nm.

8. A wastewater measurement arrangement (10) comprising an immersion probe (21) which is immersed into wastewater (13) of a wastewater tank (12), the immersion probe (21) comprising the multi-wavelength process photometer (20) of one of the preceding claims.
